# EUROPÄISCHE PATENTSCHRIFT

(11) **EP 2 236 227 B1**
(45) Veröffentlichungstag und Bekanntmachung des Hinweises auf die Patenterteilung: **18.12.2013**
(21) Anmeldenummer: 10156343.5
(22) Anmeldetag: 12.03.2010
(51) Int. Cl.: B21D 15/06, B21D 39/04, B21D 17/04, B21D 51/24, B21D 51/26, B21D 51/32, A61M 15/00, B05B 11/00, B65D 83/00, B21D 51/54

(54) **Umformwerkzeug mit einem rotierbaren Grundkörper**
Forming tool with a rotatable base body
Outil de formage doté d'un corps de base rotatif

(30) Priorität: 30.03.2009 EP 09156716
(43) Veröffentlichungstag der Anmeldung: 06.10.2010
(73) Patentinhaber: Boehringer Ingelheim International GmbH, 55216 Ingelheim am Rhein (DE)
(72) Erfinder: Spreitzer, Joerg, 55216 Ingelheim am Rhein (DE); Huebner, Michael, 55216 Ingelheim am Rhein (DE); Mathe, Gerald, 55216 Ingelheim am Rhein (DE)
(74) Vertreter: Simon, Elke Anna Maria

(56) Entgegenhaltungen:
- EP-A1- 1 025 923
- EP-A2- 0 642 992
- EP-A2- 0 916 428
- WO-A1-82/00785
- WO-A1-03/059547
- WO-A1-2009/006137
- WO-A2-2008/146025
- FR-A1- 2 505 688
- GB-A- 2 355 252
- JP-A- 2005 144 459
- US-A- 3 953 995

## Beschreibung

Die Erfindung bezieht sich auf ein Umformwerkzeug, das einen rotierbaren Grundkörper mit mindestens einer auf einer Kreisbahn angeordneten und um eine Rotationsachse drehbaren, profilierten Biegerolle umfasst.

Für die Applikation inhalationsfähiger pharmazeutischer Wirkstoffformulierungen finden vom Patienten in der Hand zu haltende und von Hand zu betätigende Inhalatoren Verwendung, in denen die pharmazeutische Wirkstoffformulierung jeweils in einer Inhalatorkartusche enthalten ist. Die Inhalatorkartusche besteht aus einer außenseitigen Aluminiumkartusche und einem darin eingesetzten Kunststoffbehälter, bei dem es sich um einen im Koextrusionsverfahren hergestellten Kunststoffbehälter handelt, der einen steifen Außenbehälter und einen darin angeordneten flexiblen Innenbeutel umfasst. Um zwischen dem Innenbeutel und dem Außenbehälter eine Druckausgleichsöffnung zu schaffen, wird beispielsweise mit dem so genannten Cut- Crack-Open Prozess eine Öffnung in den relativ steifen Außenbehälter eingebracht. Der Kunststoffbehälter wird mit der pharmazeutischen Wirkstoffformulierung gefüllt und verschlossen und im Wege des Herstellungsprozesses zur Ausbildung der Inhalatorkartusche in die Aluminiumkartusche eingesetzt. In einem folgenden Schritt wird dann im oberen Bereich der derart zusammengesetzten Inhalatorkartusche ein verformter Wandbereich in der Aluminiumkartusche ausgebildet, der sich an die Außenfläche des in die Aluminiumkartusche eingesetzten Kunststoffbehälters anlegt. Zur Ausbildung des Verbindungsbereiches am Oberrand der Aluminiumkartusche wird ein Ziehverfahren angewendet, bei dem ein rotierendes Ziehwerkzeug mit seiner Werkzeugarbeitsöffnung von oben über den auszubildenden Verbindungsbereich der Aluminiumkartusche fährt und dabei rund um die Werkzeugarbeitsöffnung herum angeordnete, profilierte Ziehrollen in Anlage mit der Mantelfläche der Aluminiumkartusche bringt, um durch eine Axialbewegung den Verbindungsbereich auszuformen. Anschließend wird die Aluminiumkartusche in einem weiteren Fertigungsschritt gasdicht an die Außenseite des darin angeordneten Kunststoffbehälters, der mit einer pharmazeutischen Wirkstoffformulierung befüllt ist, anlegt.

Bei einem aus der Praxis bekannten Umformwerkzeug, das einen rotierbaren Grundkörper mit auf einer Kreisbahn angeordneten und um eine Rotationsachse drehbaren, profilierten Biegerollen umfasst, werden die Biegerollen in einer radialen Schwenkbewegung in einer Ebene aus einer maximalen Werkzeugarbeitsöffnung in eine minimale Werkzeugarbeitsöffnung verlagert. Dieses Umformwerkzeug hat sich als nachteilig in seiner Zuverlässigkeit bzw. Störanfälligkeit erwiesen.

EP 0 916 428 betrifft in diesem Zusammenhang ein Verfahren und eine Vorrichtung zum Bilden eines Endabschnitts einer Hülse. Gemäss EP 0 916 428 ist eine Rolle bewegbar gelagert, so dass sie radial zu einer Hauptwelle hin und von ihr weg bewegt werden kann. Das Verfahren erlaubt, eine sich verjüngende Öffnung einer Hülse auszubilden.

Es ist Aufgabe der Erfindung, ein Umformwerkzeug der eingenannten Art zu schaffen, das zuverlässig arbeitet.

Erfindungsgemäß wird die Aufgabe durch das Umformwerkzeug gemäß Anspruch 1 gelöst.

Da das Umformwerkzeug rotiert, kann das Werkstück, die mit einer flüssigen Wirkstoffformulierung gefüllte, fertig zu stellende Inhalatorkartusche, fixiert werden, wodurch ein Aufschäumen der Wirkstoffformulierung vermieden ist. Das Umformwerkzeug wird zu dem Werkstück positioniert, wobei sich die Biegerolle in der die maximale Werkzeugarbeitsöffnung definierenden Position befindet. Über eine Zustellbewegung mittels des Hebels wird die Biegerolle in die die minimale Werkzeugarbeitsöffnung definierende Position verlagert. Die profilierte Biegerolle ist an die zu fertigende Kontur angepasst und durch die radiale Zustellbewegung wird beispielsweise ein umlaufender Radius an einer Oberkante des selbstverständlich zylindrischen Werkstücks rotationssymmetrisch gefertigt, wobei nach einer anfangs vorzunehmenden Einstellung bzw. Bestimmung des Durchmessers der minimalen Werkzeugarbeitsöffnung eine zuverlässige, form- und maßgenaue Fertigung zu erzielen ist. Die Rückstellung der Biegerolle aus der minimalen Werkzeugarbeitsöffnung in die maximale Werkzeugarbeitsöffnung erfolgt mittels einer radial wirksamen Druckfeder.

Nach einer Weiterbildung ist im Zentrum ein in axialer Richtung wirkender Niederhalter für das eingespannte Werkstück vorgesehen und ein zumindest abschnittweise konisches Druckstück relativ zu dem Niederhalter in axialer Richtung federbelastet verlagerbar, wobei das Druckstück mit einem Ende des Hebels zusammenwirkt, um die Biegerolle in die minimale Werkzeugarbeitsöffnung zu verlagern. Bei einer axialen Abwärtsbewegung des rotierenden Umformwerkzeuges trifft zunächst der Niederhalter auf das Werkstück, das aus einer Aluminiumkartusche mit einem darin eingesetzten Kunststoffbehälter besteht. Der Niederhalter drückt den Kunststoffbehälter in die Aluminiumkartusche und hält das Werkstück. Damit ist der Niederhalter für die Positionierung des Kunststoffbehälters in der Aluminiumkartusche zuständig. In dieser Höhenlage, in der der Niederhalter das Werkstück in axialer Richtung fixiert, befindet sich auch die Biegerolle in der erforderlichen Höhenposition. Bei einer weiteren Abwärtsbewegung wird das Druckstück nach unten verlagert und der Konus des Druckstückes beaufschlagt den Hebel, um die Biegerolle in radialer Richtung in die Position der minimalen Werkzeugarbeitsöffnung zu verlagern, in der das Werkstück seine Endkontur aufweist. Bei einer fehlerhaften axialen Positionierung würde das Druckstück den Hebel zu früh oder zu spät beaufschlagen was eine fehlerhafte, insbesondere nicht gasdichte, Endkontur zur Folge hätte.

Zur Umformung der axialen Bewegung des Druckstückes in die radiale Verlagerungsrichtung der Biegerolle ist erfindungsgemäss der Hebel an dem dem Druckstück zugewandten Ende mit einer auf dem Druckstück abrollenden Rolle versehen und an seinem gegenüberliegenden Ende schwenkbar mit dem Schieber verbunden.

Damit die Biegerolle in einer geradlinigen Bewegung in Eingriff mit dem Werkstück kommt, ist erfindungsgemäss ein fixierbarer Exzenterbolzen zum einen in eine Bohrung des Schiebers und zum anderen in ein Langloch des Hebels eingesetzt. Um in einer Feinabstimmung die minimale Werkzeugarbeitsöffnung festlegen zu können, ist der Exzenterbolzen vorgesehen. Durch dessen Verdrehung wird der Schieber mit der Biegerolle bei gleicher Lage des Druckstückes in Richtung des Werkstückes oder in entgegengesetzter Richtung verlagert. Ist die mit der Biegerolle zu fertigende Kontur anforderungsgerecht ausgeformt, wird der Exzenterbolzen beispielsweise mittels auf ihn einwirkender Schrauben festgelegt. Der Exzenterbolzen dient demnach zur radialen Einstellung der Biegerolle. Wenn die radiale Lage der Biegerolle beispielsweise durch eine Veränderung des Durchmessers der dem Hebel zugeordneten Rolle, die sich auf dem Druckstück abwälzt, bestimmt wird, ist es nicht erforderlich, einen Exzenterbolzen vorzusehen.

Um eine definierte Umlenkung der jeweiligen Bewegungsrichtung zuverlässig und reproduzierbar zu erzielen, ist vorzugsweise der Hebel schwenkbar an einem Trägerteil gelagert. Das Trägerteil ist in axialer Richtung fest zu dem Schieber positioniert und das Druckstück relativ zu dem Trägerteil verlagerbar.

Wenn sich die Biegerolle in ihrer radialen Endlage, in der sie die minimale Werkzeugarbeitsöffnung beschreibt und das Werkstück sein Fertigmaß erreicht hat, befindet, ist eine Ausweichbewegung erforderlich, um eine weitere Zustellbewegung der Biegerolle und eine damit verbundene Zerstörung des Werkstücks zu vermeiden. Nach einer Weiterbildung weist der Hebel zwei gelenkig miteinander verbundene Arme auf, zwischen denen eine federbelastete Überlastsicherung wirkt. Die Überlastsicherung bewirkt in einer radialen Lage der Beigerolle in der das Fertigmaß des Werkstückes erzielt ist, dass die beiden Arme des Hebels entgegen der Wirkung einer Feder verschwenken, um eine weitere radiale Verlagerung der Biegerolle und eine damit einhergehende Beschädigung des Werkstückes zu vermeiden. Mit der gefederten Überlastsicherung lässt sich aber auch ein zur Sicherstellung der Umformung erforderlicher Druck erzeugen und ein Ausgleich von Toleranzen des Werkstücks ist möglich. Die Feder der Überlastsicherung kann sowohl bei einem Untermaß des Werkstücks als auch bei einem Übermaß wirksam sein und spannt stets die Biegerolle gegen das Werkstück vor.

In Ausgestaltung ist auf einer Achse des Schiebers neben der Biegerolle mindestens eine Umformrolle zur Fertigung einer Sicke drehbar gelagert. Zweckmäßigerweise ist der Schieber mittels einer Nut-Feder-Führung gleitend in einem Führungsteil gelagert, das mit dem Trägerteil verbunden ist.

Zur wiederholbaren Einstellung der Höhenlage des Umformwerkzeuges mit einfachen Mitteln wirkt vorteilhafterweise eine Kurvenrolle mit einer Kurvensteuerung zur Axialbewegung des Umformwerkzeuges zusammen. Über die Steuerkurve der Kurvensteuerung können in bekannter Weise die Verfahrgeschwindigkeiten und -wege festgelegt werden. Zweckmäßigerweise sind drei radial verlagerbare Schieber mit jeweils einem Hebel sternförmig zueinander angeordnet. Mit den äquidistant verteilten Biege- und Umformrollen lässt sich das Werkstück relativ gleichmäßig verformen und es ist eine hohe Oberflächengüte unter Einhaltung verhältnismäßig enger Form- und Maßtoleranzen bei einer hohen Umformgeschwindigkeit realisierbar.

Eine Halteeinrichtung fixiert das aus einer Aluminiumkartusche und einem Kunststoffbehälter bestehende Werkstück koaxial zu einer Längsachse, entlang der das Umformwerkzeug vertikal verfährt. Die Halteeinrichtung kann Bestandteil einer Förder- und/oder Verpackungsanlage, beispielsweise in Form einer Rundtellermaschine oder eines Förderbandes, sein.

Das zuvor erläuterte Umformwerkzeug wird erfindungsgemäss zur Profilierung eines Halsbereichs einer Inhalatorkartusche verwendet, die aus einer außenseitigen Aluminiumkartusche und einem darin eingesetzten, eine Wirkstoffformulierung aufnehmenden Kunststoffbehälter besteht. Der Kunststoffbehälter kann im Koextrusionsverfahren hergestellt sein und einen steifen Außenbehälter sowie einen darin angeordneten flexiblen Innenbeutel umfassen. Der Kunststoffbehälter wird im Laufe der Herstellung mit der pharmazeutischen Wirkstoffformulierung gefüllt und verschlossen.

Die mit dem Umformwerkzeug gefertigte Inhalatorkartusche ist als Vorratsbehälter in einem Zerstäuber zur Abgabe einer bestimmten Menge eines, insbesondere ein Arzneimittel aufweisenden, Fluids als Aerosol durch eine Düse aus einem Druckspeicher eingesetzt, wobei ein mechanischer Druckerzeuger das abgemessene Fluid in dem Druckspeicher beaufschlagt, der schlagartig zum Zerstäuben freizugeben ist. Ein bekannter Zerstäuber wird unter dem Handelsnamen "Respimat" von der Boehringer Ingelheim KG in Form eines Inhalators angeboten und ist in der WO 91/14468 A1 sowie der WO 97/12687 A1 dargestellt.

Es versteht sich, dass die vorstehend genannten und nachstehend noch zu erläuternden Merkmale nicht nur in der jeweils angegebenen Kombination, sondern auch in anderen Kombinationen verwendbar sind. Der Rahmen der Erfindung ist nur durch die Ansprüche definiert.

Die Erfindung wird im Folgenden anhand zweier Ausführungsbeispiele unter Bezugnahme auf die zugehörigen Zeichnungen näher erläutert. Es zeigt:
- Fig.1: eine perspektivische Darstellung eines erfindungs- gemäßen Umformwerkzeuges,
- Fig.2: eine Ansicht des Umformwerkzeuges nach Fig. 1 von unten,
- Fig.3: eine schematische Teildarstellung des Umformwerk- zeuges nach Fig. 1,
- Fig.4: eine Teildarstellung des Umformwerkzeuges nach Fig. 1 im Schnitt,
- Fig.5: eine weitere Darstellung des Umformwerkzeuges nach Fig. 4 im Schnitt und

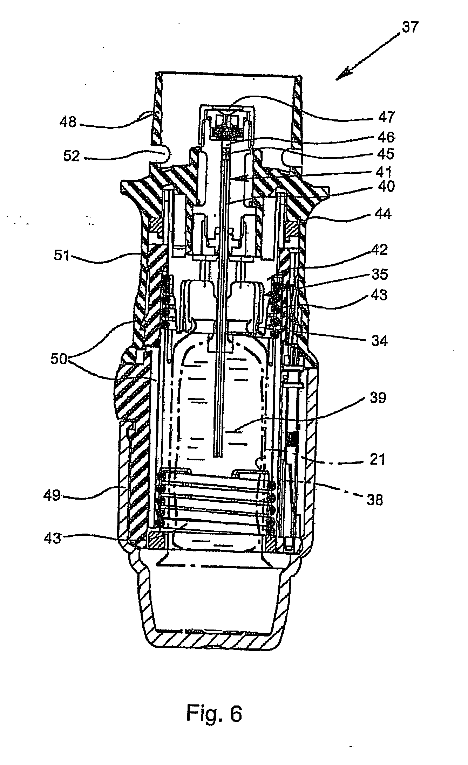

- Fig.6: eine Darstellung eines Zerstäubers mit einer mit dem Umformwerkzeug nach Fig. 1 hergestellten Inha- latorkartusche im Schnitt.

Das Umformwerkzeug umfasst eine in einer Hohlwelle 1 gelagerte Stange 2, die mit einem nicht näher dargestellten Rotationsantrieb verbunden ist. Über eine radial angebrachte werkzeugseitige Kurvenrolle 3, die mit einer Kurvensteuerung zusammenwirkt, wird eine axiale Auf- und Abbewegung des Umformwerkzeuges gesteuert. In einem Gehäuse 4 befinden sich verschiedene Lager-, Kupplungs- und Federelemente.

Unterhalb des Gehäuses 4 ist ein Druckstück 5 mit einem konischen Bereich 6 befestigt und unterhalb des Druckstückes 5 befinden sich ein Trägerteil 7 und ein Führungsteil 8, wobei in dem Trägerteil 7 drei Hebel 9 schwenkbar gelagert sind, die an einem Ende mit dem Druckstück 5 zusammenwirkende Rollen 10 aufweisen und mit den den Rollen 10 gegenüberliegenden Enden in dem Führungsteil 8 gelagerte Schieber 11 beaufschlagen. Jeder der Schieber 11 trägt auf einer Achse 12 eine Biegerolle 13 und eine Umformrolle 14.

Über die verschwenkbaren Hebel 9 sind die Biegerollen 13 aus einer eine maximale Werkzeugarbeitsöffnung definierenden Position in eine eine minimale Werkzeugarbeitsöffnung definierende Position bewegbar. Im Einzelnen drückt zunächst ein Niederhalter 15 bei einer Abwärtsbewegung des rotierenden Umformwerkzeuges gemäß dem Pfeil 16 auf ein Werkstück 17, nämlich eine Inhalatorkartusche 36. Bei einer weitergehenden Abwärtsbewegung werden die Hebel 9 gemäß dem Pfeil 18 aufgrund der Wirkung des konischen Bereichs 6 des Druckstückes 5 verschwenkt und die Schwenkbewegung des Hebels 9 wird in eine radiale Zustellbewegung des Schiebers 11 gemäß dem Pfeil 19 umgeformt, um die Biegerolle 13 und die Umformrolle 14 in ihre Endlage, in der sie die minimale Werkzeugarbeitsöffnung beschreiben, zu verfahren. Wird das Umformwerkzeug in axialer Richtung nach oben verfahren, werden die Hebel 9 mittels radial wirkender Druckfedern, die sich an einem Zwischenring 20 abstützen, derart verschwenkt, dass die Biegerolle 13 und die Umformrolle 14 in eine Position verlagert werden, in der sie die maximale Werkzeugarbeitsöffnung beschreiben.

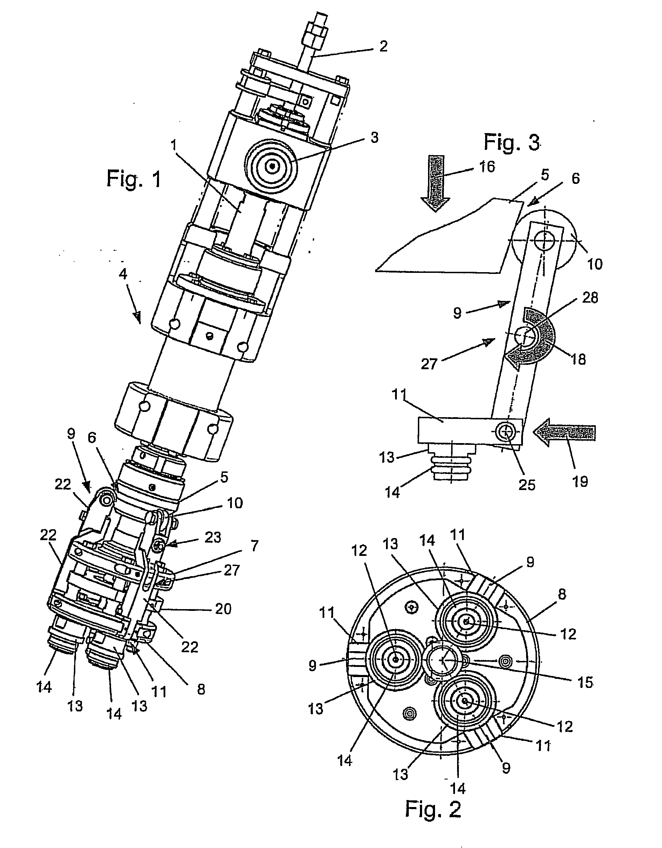

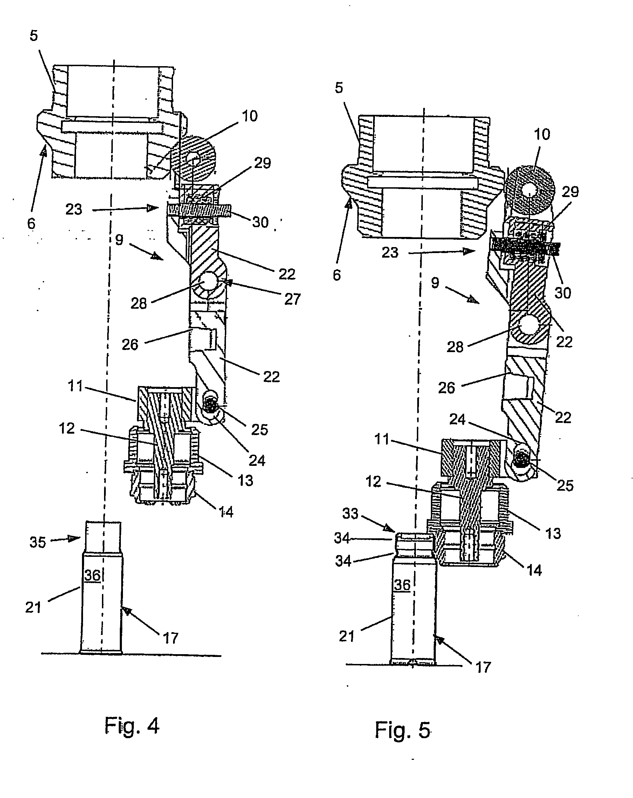

Um eine Beschädigung des aus einer Aluminiumkartusche 21 mit einem darin eingesetzten Kunststoffbehälter 38 bestehenden Werkstückes 17 zu vermeiden, umfasst der Hebel 9 gemäß den Fig. 1, 4 und 5 zwei gelenkig miteinander verbundene Arme 22, zwischen denen eine federbelastete Überlastsicherung 23 wirkt. Der erste Arm 22 des Hebels 9 weist ein Langloch 24 auf, das ein mit dem Schieber 11 fixierbar verbundener Exzenterbolzen 25 durchragt. Im Weiteren ist in diesen Arm 22 eine Sackbohrung 26 für eine radial wirkende Rückstellfeder eingelassen. Der erste Arm 22 und der zweite Arm 22 des Hebels 9 stehen über ein gemeinsames Schwenkgelenk 27 mit einem in das Trägerteil 7 eingreifenden Stift 28 in Verbindung. Im Bereich unterhalb der dem Druckstück 5 zugeordneten Rolle 10 ist eine Feder 29 derart auf einem Führungsstift 30 angeordnet, dass der zweite Arm 22 gegenüber dem ersten Arm 22 des Hebels 9 entgegen der Wirkung der Feder 29 schwenkbar ist, um die Biegerolle 13 gegenüber dem Werkstück 17 vorzuspannen und Toleranzen des Werkstücks 17 auszugleichen. Insbesondere ist der zweite Arm 22 gegenüber dem ersten Arm 22 des Hebels 9 entgegen der Wirkung der Feder 29 schwenkbar, wenn die Biegerolle 13 und die Umformrolle 14 ihre Endlage erreicht haben und das Druckstück 5 weitere Kraft auf den Hebel 9 ausübt.

Mit den Biegerollen 13 wird im umfangsseitigen Randbereich des Werkstückes 17 ein Radius 33 geformt, wobei der freie Rand der Aluminiumkartusche 21 derart geformt wird, dass er auf der freien Stirnseite des Kunststoffbehälters aufliegt. Die Umformrollen 14 dienen im Wesentlichen zur Ausformung von zwei umlaufenden rillenförmigen Sicken 34 in einem gezogenen Halsbereich 35 des Werkstückes 17.

Die Inhalatorkartusche 36 findet Verwendung in einem Zerstäuber 37, der zur Zerstäubung eines Fluids 39 dient, insbesondere eines hochwirksamen Arzneimittels, und als tragbarer Inhalator ausgebildet ist, der ohne Treibgas arbeitet. Bei der Zerstäubung des Fluids 2, vorzugsweise einer Flüssigkeit, wird ein Aerosol gebildet, das von einem nicht dargestellten Benutzer eingeatmet werden kann.

Der Zerstäuber 37 weist die Inhalatorkartusche 36 mit dem Fluid 39 auf, die von unten in den geöffneten Zerstäuber 37 einsetzbar ist. Zur Zerstäubung des Fluids 39 in einer vorbestimmten einstellbaren Menge umfasst der Zerstäuber 37 einen einen Kolben umfassenden Druckerzeuger 41 mit einer Halterung 42 für die Inhalatorkartusche 36, einer Antriebsfeder 43 mit einer zur Entspannung manuell zu betätigenden Lösetaste 44, einem Förderrohr 40 mit einem eingesetzten Rückschlagventil 45, einer Druckkammer 46 und einer Düse 47, der ein Mundstück 48 zugeordnet ist.

Beim axialen Spannen der Antriebsfeder 43 durch ein Drehen eines Gehäuseunterteils 49 mit einem daran lösbar befestigten Innenteil 50 relativ zu einem an dem Mundstück 48 angeformten Gehäuseoberteil 51 wird die Halterung 42 mit der Inhalatorkartusche 36 und dem Förderrohr 40 nach unten bewegt und Fluid 39 aus der Inhalatorkartusche 36 über das Rückschlagventil 45 in die dem Kolben des Druckerzeugers 41 zugeordnete Druckkammer 46 gesaugt. Beim anschließenden schlagartigen Entspannen der Antriebsfeder 43 durch die Betätigung der Lösetaste 44 wird das Fluid 39 in der Druckkammer 46 von der das Förderrohr 40 nach oben verlagernden Antriebsfeder 43 unter Druck gesetzt und über die Düse 47 ausgegeben, wobei eine Zerstäubung stattfindet. Die Zerstäubung erfolgt beispielsweise in Partikel im µm-Bereich, vorzugsweise in Partikel mit einer Größe von etwa 20 µm, die eine Wolke bzw. einen Strahl eines Aerosols bilden. Ein Benutzer kann das Aerosol inhalieren, wobei Zuluft über Zuluftöffnungen 52 in dem Mundstück 48 ansaugbar ist.

### Bezugszeichenliste

- 1.: Hohlwelle
- 2.: Stange
- 3.: Kurvenrolle
- 4.: Gehäuse
- 5.: Druckstück
- 6.: konischer Bereich
- 7.: Trägerteil
- 8.: Führungsteil
- 9.: Hebel
- 10.: Rolle
- 11.: Schieber
- 12.: Achse
- 13.: Biegerolle
- 14.: Umformrolle
- 15.: Niederhalter
- 16.: Pfeil
- 17.: Werkstück
- 18.: Pfeil
- 19.: Pfeil
- 20.: Zwischenring
- 21.: Aluminiumkartusche
- 22.: Arm v. 9
- 23.: Überlastsicherung
- 24.: Langloch
- 25.: Exzenterbolzen
- 26.: Sackbohrung
- 27.: Schwenkgelenk
- 28.: Stift
- 29.: Feder
- 30.: Führungsstift
31.
32.
- 33.: Radius
- 34.: Sicke
- 35.: Halsbereich
- 36.: Inhalatorkartusche
- 37.: Zerstäuber
- 38.: Kunststoffbehälter
- 39.: Fluid
- 40.: Förderrohr
- 41.: Druckerzeuger
- 42.: Halterung
- 43.: Antriebsfeder
- 44.: Lösetaste
- 45.: Rückschlagventil
- 46.: Druckkammer
- 47.: Düse
- 48.: Mundstück
- 49.: Gehäuseunterteil
- 50.: Innenteil
- 51.: Gehäuseoberteil
- 52.: Zuluftöffnungen

## Patentansprüche

1. Umformwerkzeug, das einen rotierbaren Grundkörper mit mindestens einer auf einer Kreisbahn angeordneten und um eine Rotationsachse drehbaren, profilierten Biegerolle (13) umfasst, wobei die an einem radial verlagerbaren Schieber (11) angeordnete Biegerolle (13) aus einer eine maximale Werkzeugarbeitsöffnung definierenden Position über einen federbelasteten Hebel (9), der einen axialen Verlauf aufweist, in eine eine minimale Werkzeugarbeitsöffnung definierende Position bewegbar ist, **dadurch gekennzeichnet, dass** der Hebel (9) an dem einem Druckstück (5) zugewandten Ende mit einer auf dem Druckstück (5) abrollenden Rolle (10) versehen und an seinem gegenüberliegenden Ende schwenkbar mit dem Schieber (11) verbunden ist, und wobei ein fixierbarer Exzenterbolzen (25) zum einen in eine Bohrung des Schiebers (11) und zum anderen in ein Langloch (24) des Hebels (9) eingesetzt ist.

2. Umformwerkzeug nach Anspruch 1, **dadurch gekennzeichnet, dass** im Zentrum ein in axialer Richtung wirkender Niederhalter (15) für das eingespannte Werkstück (17) vorgesehen und ein zumindest abschnittweise konisches Druckstück (5) relativ zu dem Niederhalter (15) in axialer Richtung federbelastet verlagerbar ist, wobei das Druckstück (5) mit einem Ende des Hebels (9) zusammenwirkt, um die Biegerolle (13) in die minimale Werkzeugarbeitsöffnung zu verlagern.

3. Umformwerkzeug nach Anspruch 1 oder 2, **dadurch gekennzeichnet, dass** der Hebel (9) schwenkbar an einem Trägerteil (7) gelagert ist.

4. Umformwerkzeug nach einem der Ansprüche 1 bis 3, **dadurch gekennzeichnet, dass** der Hebel (9) zwei gelenkig miteinander verbundene Arme (22) aufweist, zwischen denen eine federbelastete Überlastsicherung (23) wirkt.

5. Umformwerkzeug nach Anspruch 1, **dadurch gekennzeichnet, dass** auf einer Achse (12) des Schiebers (11) neben der Biegerolle (13) mindestens eine Umformrolle (14) zur Fertigung einer Sicke (34) drehbar gelagert ist.

6. Umformwerkzeug nach einem der Ansprüche 1 bis 5, **dadurch gekennzeichnet, dass** der Schieber (11) mittels einer Nut-Feder-Führung gleitend in einem Führungsteil (8) gelagert ist, das mit dem Trägerteil (7) verbunden ist.

7. Umformwerkzeug nach Anspruch 1, **dadurch gekennzeichnet, dass** eine Kurvenrolle (3) mit einer Kurvensteuerung zur Axialbewegung des Umformwerkzeuges zusammenwirkt.

8. Umformwerkzeug nach Anspruch 1, **dadurch gekennzeichnet, dass** drei radial verlagerbare Schieber (11) mit jeweils einem Hebel (9) sternförmig zueinander angeordnet sind.

9. Verwendung eines Umformwerkzeuges nach Anspruch 1 zur Profilierung eines Halsbereichs (35) einer Inhalatorkartusche (36), die aus einer außenseitigen Aluminiumkartusche (21) und einem darin eingesetzten, eine Wirkstoffformulierung aufnehmenden Kunststoffbehälter (38) besteht.

## Claims

1. Shaping tool which comprises a rotatable base member having at least one profiled bending roller (13) which is mounted on a circular track and is rotatable about a rotation axis, the bending roller (13) arranged on a radially movable slide (11) being movable from a position that defines a maximum working aperture for the tool, by means of a spring-loaded lever (9) with an axial configuration, into a position that defines a minimum working aperture for the tool, **characterised in that** the lever (9) is provided, at its end facing a pressing member (5), with a roller (10) that rolls on the pressing member (5) and at its opposite end it is pivotably connected to the slide (11), and wherein a fixable eccentric bolt (25) is inserted in a bore in the slide (11), on the one hand, and in an oblong hole (24) in the lever (9), on the other.

2. Shaping tool according to claim 1, **characterised in that** in the centre, an axially acting depressor (15) for the clamped workpiece (17) is provided and a pressing member (5) that is conical at least in parts is axially movable under spring loading relative to the depressor (15), while the pressing member (5) cooperates with one end of the lever (9) in order to move the bending roller (13) into the minimum working aperture for the tool.

3. Shaping tool according to claim 1 or 2, **characterised in that** the lever (9) is pivotably mounted on a carrier member (7).

4. Shaping tool according to one of claims 1 to 3, **characterised in that** the lever (9) has two arms (22) articulated to one another between which a spring-loaded overload prevention device (23) acts.

5. Shaping tool according to claim 1, **characterised in that** at least one shaping roller (14) for producing a crimp (34) is rotatably mounted on a spindle (12) of the slide (11) adjacent to the bending roller (13).

6. Shaping tool according to one of claims 1 to 5, **characterised in that** the slide (11) is slidably mounted by means of a tongue and groove guide in a guide portion (8) that is connected to the carrier member (7).

7. Shaping tool according to claim 1, **characterised in that** a cam roller (3) cooperates with a cam control for the axial movement of the shaping tool.

8. Shaping tool according to claim 1, **characterised in that** three radially adjustable slides (11) each having one lever (9) are arranged in a star shape with one another.

9. Use of a shaping tool according to claim 1 for profiling a neck region (35) of an inhaler cartridge (36), which consists of an external aluminium cartridge (21) and a plastic container (38) inserted therein holding an active substance formulation.

## Revendications

1. Outil de formage, qui comprend un corps de base rotatif doté d'au moins un galet de cintrage (13) profilé, disposé sur une trajectoire circulaire et pouvant tourner autour d'un axe de rotation, le galet de cintrage (13), disposé au niveau d'une coulisse (11) déplaçable radialement, pouvant être déplacé depuis une position définissant une ouverture maximale de travail d'outil dans une position définissant une ouverture minimale de travail d'outil par l'intermédiaire d'un levier (9) soumis à l'action d'un ressort et présentant un tracé axial, **caractérisé en ce que** le levier (9) est pourvu, au niveau de l'extrémité tournée vers une pièce de pression (5), d'un galet (10) roulant sur la pièce de pression (5) et est relié, au niveau de son extrémité opposée, de manière à pouvoir pivoter, à la coulisse (11), et un boulon excentrique (25) fixable étant inséré d'une part dans un alésage de la coulisse (11) et d'autre part dans un trou oblong (24) du levier (9).

2. Outil de formage selon la revendication 1, **caractérisé en ce qu'**est prévu au centre un serreflan (15) agissant dans une direction axiale pour la pièce à usiner (17) serrée, et **en ce qu'**une pièce de pression (5) conique au moins par endroits peut être déplacée par rapport au serreflan (15) sous l'action d'un ressort dans une direction axiale, la pièce de pression (5) coopérant avec une extrémité du levier (9) pour déplacer le galet de cintrage (13) dans l'ouverture minimale de travail d'outil.

3. Outil de formage selon la revendication 1 ou 2, **caractérisé en ce que** le levier (9) est logé de manière à pouvoir pivoter au niveau d'une partie de support (7).

4. Outil de formage selon l'une quelconque des revendications 1 à 3, **caractérisé en ce que** le levier (9) présente deux bras (22) reliés entre eux de manière articulée, entre lesquels agit une sûreté anti-surcharge (23) soumise à l'action d'un ressort.

5. Outil de formage selon la revendication 1, **caractérisé en ce qu'**au moins un galet de formage (14) servant à produire une moulure (34) est logé de manière à pouvoir tourner sur un axe (12) de la coulisse (11) à proximité du galet de cintrage (13).

6. Outil de formage selon l'une quelconque des revendications 1 à 5, **caractérisé en ce que** la coulisse (11) est logée de manière coulissante dans une partie de guidage (8) au moyen d'un guidage à rainure et languette, ladite partie de guidage étant reliée à la partie de support (7).

7. Outil de formage selon la revendication 1, **caractérisé en ce qu'**un galet de came (3) coopère avec une commande de came afin de déplacer axialement l'outil de formage.

8. Outil de formage selon la revendication 1, **caractérisé en ce que** trois coulisses (11) déplaçables radialement et respectivement dotées d'un levier (9) sont disposées les unes par rapport aux autres de manière à présenter une forme d'étoile.

9. Utilisation d'un outil de formage selon la revendication 1 servant à profiler une zone de goulot (35) d'une cartouche d'inhalateur (36) qui est constituée d'une cartouche en aluminium (21) côté extérieur et d'un récipient en plastique (38) inséré dans ladite cartouche et recevant une formulation de principe actif.
